**Europäisches Patentamt**

**European Patent Office**

**Office européen des brevets**

(11) Veröffentlichungsnummer: **0 150 352**
**B1**

(12) **EUROPÄISCHE PATENTSCHRIFT**

(45) Veröffentlichungstag der Patentschrift:
29.06.88

(51) Int. Cl.⁴: **A 61 B 5/04**

(21) Anmeldenummer: **84114875.2**

(22) Anmeldetag: **06.12.84**

(54) Verfahren zur Bestimmung des Anfangs- und Endpunktes von geschlossenen, physiologischen Messsignalen.

(30) Priorität: **25.01.84 CH 327/84**

(43) Veröffentlichungstag der Anmeldung:
**07.08.85 Patentblatt 85/32**

(45) Bekanntmachung des Hinweises auf die Patenterteilung:
**29.06.88 Patentblatt 88/26**

(84) Benannte Vertragsstaaten:
**AT BE CH DE FR GB IT LI NL SE**

(56) Entgegenhaltungen:
EP-A-0 052 512
EP-A-0 086 429

BIOMEDIZINISCHE TECHNIK, Band 23, Heft 1-2, Januar 1978, Seiten 2-8, Berlin, DE; R.-D. BÖCKMANN u.a.: "Neue Vermessungsalgorithmen zur Bestimmung von QRS-Anfang und -Ende im Kinder-EKG und -VKG"
MEDICAL & BIOLOGICAL ENGINEERING AND COMPUTING, Band 18, Nr. 3, Mai 1980, Seiten 304-311, Stevenage, GB; I.-C. CHIEN u.a.: "Computer methods for analysing the high-frequency electrocardiogram"
J. PÄTZOLD: "Kompendium Elektromedizin, Grundlagen, Technik, Anwendungen", 1976, Seiten 26-30, Siemens, Berlin, DE;
COMPUTER, Band 18, nr. 7, Juli 1975, Seiten 42-45, Long Beach, US; M.L. SIMOONS et al.: "On-line

(73) Patentinhaber: **WILLI STUDER AG Fabrik für elektronische Apparate, Althardstrasse 30, CH- 8105 Regensdorf ZH (CH)**

(72) Erfinder: **Thie, Werner, Sandbuckweg 9, CH- 8157 Dielsdorf ZH (CH)**
Erfinder: **Schmid, Johann Jakob, Rosengartenstrasse 33, CH- 8107 Buchs ZH (CH)**

(56) Entgegenhaltungen: (Fortsetzung)
analysis of exercise electrocardiograms"
WESCON TECHNICAL PAPERS, Band 19, Western electronic show and convention, 16.-19. September 1975, Seiten 1-6, Artikel Nr. 24/3; R.V. RAMASWAMI: "The microcomputer in diagnostic health care and patient monitoring"

EP 0 150 352 B1

## Beschreibung

Die Erfindung bezieht sich auf ein Verfahren zur Bestimmung des Anfangs- und Endpunktes von sich wiederholenden Vorgängen, im Körper eines Lebewesens, die durch Meßwerte die mittels Elektroden abgegriffen werden in Funktion von Raum und Zeit dargestellt werden.

Solche Vorgänge werden durch an sich bekannte Verfahren zur Vektorkardiographie dargestellt. Ein solches Verfahren ist in der europäischen Patentanmeldung Nr. 0 086 429 ausfuehrlich beschrieben. Bei einem solchen, wie auch bei anderen bekannten Verfahren der vektorkardiographie, werden Anfangs- und Endpunkte beispielsweise der P-, QRS- und T-Wellen durch den Arzt, der die vorliegenden Kurven interpretieren muß, visuell bestimmt. Dies geht beispielsweise aus "Computers in Cardiology 1982 IEEE", Seite 429 ff: "Automated vectorcardiographic analysis by an inexpensive microprocessor" (ISBN No. 0-8186-0024-1) hervor. Daraus geht ebenfalls hervor, daß die Bestimmung der Anfangs- und Endpunkte durch Berechnung in einem elektronischen Rechner eher noch weniger zuverlässige Resultate liefert, als die einfache visuelle Bestimmung.

Aus "Biomedizinische Technik" Band 23, Heft 1-2, Januar 1978, Seiten 2-8) R. D. Boeckmann: "Neue Vermeßungsalgorithmen zur Bestimmung von QRS-Anfang und Ende im Kinder EKG und VKG" ist es bekannt, aus statistisch ermittelten Erwartungswerten für die räumlich vektorielle Geschwindigkeit des zu vermessenden QRS-Komplexes eine Schablone zu bilden. Der Vergleich der vorliegenden Werte mit der Schablone erlaubt die Bestimmung von QRS-Anfang und QRS-Ende.

Eine weitere Möglichkeit zur Bestimmung der Anfangs- und Endpunkte eines Vorganges wie ihn ein bekanntes Vektorkardiogramm darstellt, besteht darin, in der vorliegenden Darstellung Teilstücke herauszusuchen, bei denen die Meßwerte sich zeitlich nicht oder fast nicht ändern. Solchen Teilstücken kann ein elektrisches Anfangs End- oder Basispotential zugesprochen werden, was darauf hindeutet, daß die Anfangs- und Endpunkte des Vorganges im Bereiche dieser Teilstücke liegen. Nach wie vor unbestimmt ist bei einem solchen Verfahren die zeitliche Definition der Anfangs und Endpunkte.

Besonders schwierig ist die Bestimmung von Anfangs und Endpunkten eines Vorganges dann, wenn diesem sehr niederfrequente Schwingungen überlagert sind. Bei einem Vektorkardiogramm wird beispielsweise eine solche Schwingung durch die Athmung eines Patienten bewirkt. Wird das Vektorkardiogramm unter Belastung des Kreislaufes aufgenommen, so verzerren diese Schwingungen die Darstellung derart, daß eine Bestimmung von Anfangs- und Endpunkten durch bisher bekannte Methoden mit genügender Zuverläßigkeit unmöglich ist.

Aus der europäischen Patentanmeldung Nr 0 052 512 ist ein weiteres Verfahren zur Bestimmung des Endpunktes eines QRS-Komplexes in einer EKG-Kurve bekannt. Dabei wird die Differenz zwischen gemeßenen Potentialwerten und einem Nullwert berechnet. Dort wo die kleinste Differenz auftritt ist dann der Endpunkt zu vermuten.

Die Erfindung, wie sie in den Ansprüchen gekennzeichnet ist, löst die Aufgabe, ein Verfahren für die Bestimmung von Anfangs- und Endpunkten von Vorgängen zu schaffen, mit dem der Zeitpunkt dieser Anfangs- und Endpunkte eindeutig festgestellt werden kann.

Die durch die Erfindung erreichten Vorteile sind darin zu sehen, daß die Anfangs- und Endpunkte unabhängig von der Nullinie bestimmt werden und damit nicht durch niederfrequente Schwingungen gestört werden. Zudem kann die Größe der gemessenen Signalausschläge genau bestimmt werden, nachdem der Zeitpunkt des Anfangs und Endpunktes und daraus auch die Signalgröße in diesen Punkten bekannt ist. Die Signalgröße im Anfangs und Endpunkt ergibt eine Referenzgröße. Ebenso können verschiedene Signale, die verschiedene Vorgänge darstellen, gegeneinander genau abgegrenzt werden.

Bei einem Vektorkardiogramm, bei dem die Herztätigkeit eines Patienten an der zeitlichen Änderung des elektromagnetischen Feldes, das vom Herz erzeugt und durch einen Vektor dargestellt werden kann, beobachtet wird, wird versucht, Richtung und Betrag des Vektors zu verfolgen. Dazu wird der Vektor bzw. dessen Projektion auf mehrere Ebenen betrachtet. Dies ergibt in der Praxis mehrere zeitlich miteinander verknüpfte Signalverläufe. Nur wenn Anfangs- und Endpunkt jedes einzelnen Signalverlaufes bekannt sind, kann eine zuverlässige Diagnose und Deutung der einzelnen Signalverläufe erfolgen. Dabei sind für die Diagnose neben Betrag und Richtung des Vektors auch der Einsatzpunkt der QRS-P- und T-Welle, der Zeitpunkt des Beginns und die Länge der Intervalle dazwischen von Bedeutung. Diese sind dank dem erfindungsgemäßen Verfahren bekannt. Je schneller der Herzschlag, desto unsicherer werden die Resultate aus den bisher bekannten verfahren. Die Sicherheit des erfindungsgemäßen Verfahrens dagegen bleibt auch in diesen Fällen erhalten.

Im folgenden wird die Erfindung anhand von lediglich einen Ausführungsweg darstellenden Zeichnungen näher erläutert.

Es zeigt:

Figur 1    eine Darstellung eines, mittels Elektroden von einem Patienten erfaßten, Vorganges in einer Ebene über einer Zeitachse aufgetragen und

Figur 2    eine Darstellung eines Vorganges gemäß Figur 1 im Raum sowie in drei verschiedene Ebenen projiziert.

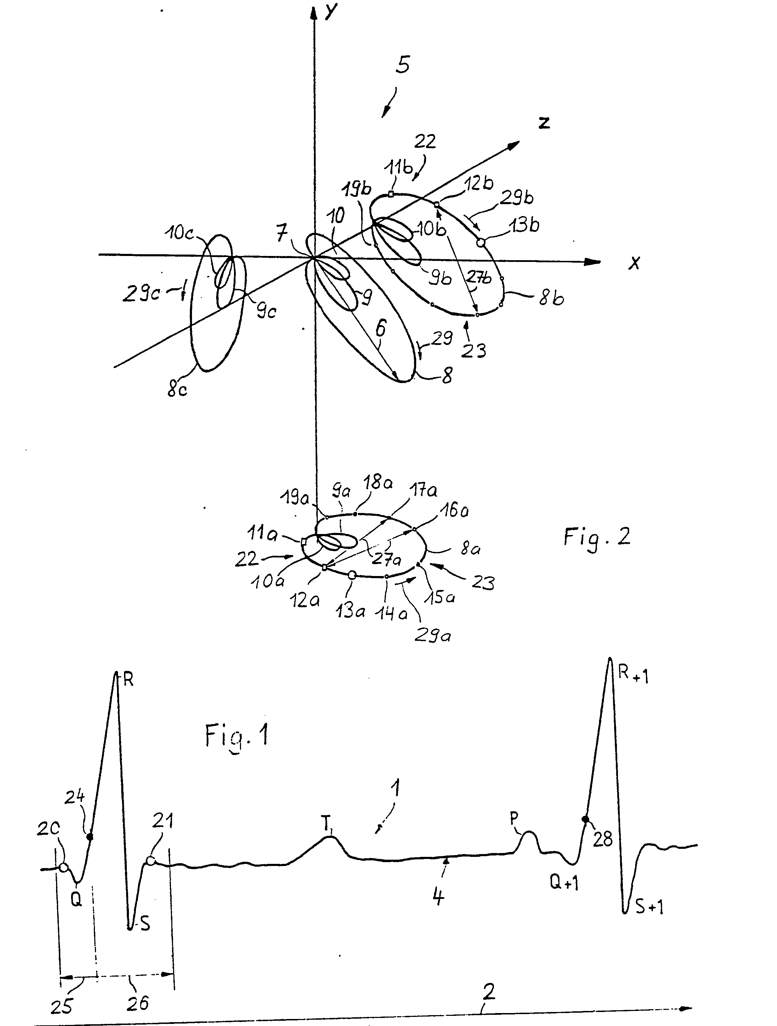

Figur 1 zeigt als Darstellung eines Vorganges, beispielsweise einen Ausschnitt aus einer EKG-Kurve 1. Dabei sind elektrische Potentialwerte, also Meßwerte die mit Elektroden von einem Lebewesen abgegriffen werden über einer Zeitachse 2 aufgetragen. Da das erfindungsgemäße Verfahren am Beispiel eines zeitlich ständig seinen Betrag und seine Richtung ändernden Vektors, der die Tätigkeit des Herzens eines Lebewesens darstellt, erklärt werden soll, können diese Potentialwerte als Betrag, des in eine Ebene projizierten Vektors aufgefaßt werden.

Ein solcher Vektor bzw. die seinem Endpunkt zugeordneten Potentialwerte beschreiben im zeitlichen Ablauf nacheinander Vorgänge, die beispielsweise als QRS-Welle, T-Welle und P-Welle dem Kardiologen bekannt sind. Zwischen diesen Wellen sind mehr oder weniger kurze und mehr oder weniger konstante Potentialwerte anzeigende Teilstrecken 4 der Kurve 1 eingeschoben.

Figur 2 zeigt in einem dreiachsigen Koordinatensystem 5 den Vektor beispielsweise zu einem bestimmten Zeitpunkt als Pfeil 6 dargestellt. Dieser Vektor geht aus von einem Nullpunkt 7 und endet in Punkten im Raum, die zusammen Schleifen 8, 9 und 10 bilden. Dabei wird die Schleife 8 auch als QRS-Schleife, die Schleife 9 als T-Schleife und die Schleife 10 als P-Schleife bezeichnet. Durch die X-, Y- und Z-Achse des Koordinatensystems 5 werden je eine XY-, XZ- und YZ-Ebene gegeben. Die Schleifen 8, 9 und 10 lassen sich in diese drei Ebenen projizieren so, daß weitere Schleifen 8a, b, c, 9a, b, c und 10a, b, c erkennbar sind. Zur Erleichterung nachfolgender Erläuterungen sind auch einzelne Punkte 11a, b bis 19a, b, längs der Schleifen 8a und 8b eingetragen.

Im folgenden werden nun die einzelnen Verfahrensschritte beschrieben, die es erlauben, Anfangs- und Endpunkte beispielsweise einer QRS-Schleife zu bestimmen. Da der Vektor jeweils nacheinander eine QRS-, eine T- und eine P-Schleife beschreibt, ist es wichtig zu wissen, wo und wann beispielsweise die QRS-Schleife beginnt und endet, denn die QRS-Schleife gibt dem Arzt Hinweise auf bestimmte Aspekte der Herztätigkeit und die T- oder die P-Schleifen geben Hinweise auf andere Aspekte der Herztätigkeit.

Um zuverlässige Angaben über die Lage von Anfangs- und Endpunkten 20 und 21 (Figur 1) der QRS-Welle oder Schleife zu erhalten, ist es unerläßlich, die Bewegungen des Vektors und somit die QRS-Schleife 8, 8a, 8b, 8c in mehreren Ebenen zu erfassen. Deshalb werden bei der Erstellung eines Vektorkardiogrammes in an sich bekannter Weise periodisch oder kontinuierlich Werte des Potentiales, das durch den Vektor dargestellt wird ermittelt. Die graphische Darstellung solcher Werte über eine Zeitachse 2 aufgetragen, ergibt eine Kurve 1. Die graphische Darstellung solcher Werte in einer XZ-, einer XY- und ZY-Ebene aufgetragen, ergibt QRS-Schleifen 8a, 8b und 8c.

Bei nicht kontinuierlicher, digitaler Erfassung der Werte des Potentiales werden zu bestimmten Abtastzeitpunkten X-Werte, Y-Werte und Z-Werte des Potentiales ermittelt und gespeichert. Eine geeignete Kombination solcher Werte von zwei verschiedenen Achsen ergibt zusammen mit den verschiedenen Abtastzeitpunkten an denen diese Werte ermittelt werden Punkte 11a bis 19a der QRS-Schleife 8a oder beispielsweise Punkte 11b bis 19b einer QRS-Schleife 8b.

Aus den Differenzen der Werte zweier aufeinanderfolgender Abtastzeitpunkte ergeben sich Steigungswerte des Vektors bzw. des Vorganges.

Dies kann getrennt für jede der drei Ebenen des Koordinaten system 5 durchgeführt werden. So wird beispielsweise ein Steigungswert für den Bereich zwischen Punkten 12a und 13a in der X-Z-Projektion, 12b und 13b in der X-Y-Projektion ermittelt usw. Dies wird mit allen abgetasteten Werten der QRS-Schleifen 8a, 8b und 8c durchgeführt und man erhält für jeden Bereich zwischen zwei aufeinanderfolgenden Punkten drei Steigungswerte. Aus allen so erhaltenen Steigungswerten wird die räumliche Steigung und aus allen ermittelten Werten für die räumliche Steigung ein Mittelwert errechnet.

Ein Vergleich der einzelnen Steigungswerte mit dem Mittelwert erlaubt es beispielsweise Steigungswerte, die den Mittelwert um ein bestimmtes Maß übersteigen auszuschließen und nicht mehr weiter zu berücksichtigen. Aus den übriggebliebenen Steigungswerten wird ein neuer Mittelwert gebildet.

Um den neuen Mittelwert wird ein Erwartungsintervall gelegt, in welchen Steigungswerte des Vektors vernünftigerweise liegen sollen. Dies, um beispielsweise beim Herzen auftretende Extrasystolen, die hohe Steigungswerte aufweisen, von den Betrachtungen auszuschließen.

Anschließend wählt man aus gleichsinnigen aufeinanderfolgenden Steigungswerten, die in dem genannten Erwartungsintervall liegen, den ersten der in dem genannten Erwartungsintervall liegt. Zu diesem Steigungswert wird der Zeitpunkt 24 ermittelt, in welchem der erste der beiden für die Berechnung der Steigung notwendigen Potentialwerte abgetastet wurde.

Dieser Zeitpunkt kann auch als Referenzpunkt bezeichnet werden und dient uns dazu, die QRS-Schleife bzw. deren Projektionen 8a, 8b, 8c in je einen ersten Abschnitt 22 und einen zweiten Abschnitt 23 aufzuteilen. In der Darstellung gemäß Figur 2 markiert der Punkt 13a, 13b den Zeitpunkt bzw. den Referenzpunkt. Dieser Zeitpunkt 24 dient als Referenzpunkt für Betrachtungen oder Messungen an der QRS-Schleife oder -Welle oder an einer ganzen Herzschlagperiode.

Vom Zeitpunkt 24, 13a, 13b wird ein erstes und ein zweites Zeitintervall 25 und 26 vorgegeben. Das Zeitintervall 25 liegt vor dem Zeitpunkt 24, das Zeitintervall 26 liegt nach dem Zeitpunkt 24 bzw. 13a, 13b usw. Für die Untersuchung am menschlichen Herz beträgt das erste Zeitintervall 25 vorzugsweise ca. 60 Millisekunden, das zweite Zeitintervall 26 maximal ca. 200 Millisekunden.

Den Zeitintervallen 25 und 26, gemäß Figur 1, entsprechen die Abschnitte 22 und 23 vor und nach dem Punkt 13a, 13b usw. in der Darstellung gemäß Figur 2. Pfeile 29, 29a, 29b, 29c geben die zeitliche Folge der Schleifen bzw. Darstellungen 8, 8a, 8b und 8c des Vorganges an.

In einem weiteren Schritt werden nun Abstände 27a, 27b usw. zwischen Punkten 11a, 12a, 11b, 12b usw. des Abschnittes 22 und Punkten 14a bis 19a, 14b bis 19b usw. des Abschnittes 23 der QRS-Schleife 8a, 8b usw. ermittelt. Die ermittelten Abstände 27a, 27b usw. zwischen Endpunkten des Vektors in den Abschnitten 22 bzw. 23 berechnen sich z. B. in einem 3-achsigen Koordinatensystem nach der bekannten Formel:

$$a = \sqrt{(X_1 - X_2)^2 + (Y_1 - Y_2)^2 + (Z_1 - Z_2)^2}$$

für zwei Punkte, welche durch Indizes 1 und 2 bezeichnet sind.

Die erhaltenen Werte der räumlichen Abstände zwischen allen möglichen Kombinationen von zwei Punkten aus den Abschnitten 22 und 23 werden nun nach dem kleinsten dieser Abstände abgesucht. Jedem dieser beiden Punkte, als die wir beispielsweise die Punkte 11 und 19 bzw. 11a und 19a bzw. 11b und 19b usw. annehmen wollen, ist ein Abtastzeitpunkt zugeordnet. Diese Abtastzeitpunkte der beiden Punkte, die den kleinsten Abstand zueinander aufweisen, werden nun als Anfangspunkt und Endpunkt 21 der QRS-Welle bzw. als Nullpunkte 7 der QRS-Schleife bezeichnet.

Das eben beschriebene Verfahren eignet sich besonders gut in den Fällen, in denen zuerst alle Daten erfaßt und anschließend ausgewertet werden. Das verfahren kann aber auch für unmittelbare laufende Datenauswertung, die schon während der Datenerfassung erfolgt, angepasst werden.

Dazu kann beispielsweise für den ersten erfaßten Herzschlag ein fester Steigungswert vorgegeben werden. Der erste ermittelte Steigungswert, der diesen vorgegebenen Steigungswert erreicht, liefert einen Abtastzeitpunkt, der als Zeitpunkt 24 oder erster Referenzpunkt angenommen wird. Ausgehend von diesem Referenzpunkt werden die Anfangs- und Endpunkte der QRS-Welle auf bereits bekannte Art ermittelt.

Für den zweiten Herzschlag der Meßreihe kann eine verbesserte Annahme für den Referenzpunkt gemacht werden, indem man nach dem Referenzpunkt des ersten Herzschlages die maximal auftretende gleichsinnige Steigung ermittelt und den dazugehörigen Abtastzeitpunkt als neuen Referenzpunkt für den zweiten Herzschlag betrachtet.

Es kann weiter die Zeitdauer zwischen den nun bekannten Referenzpunkten 24 und 28 des ersten und des zweiten Herzschlages gemessen werden und ausgehend vom Anfangs- und Endpunkt des ersten Herzschlages abgetragen werden. So erhält man Anfangs- und Endpunkt des zweiten Herzschlages.

So kann die Ermittlung des Referenzpunktes und daraus folgend die Ermittlung der Anfangs- und Endpunkte der QRS-Welle laufend verbessert werden. Als Resultat dieses Verfahrens steht für jeden Herzschlag oder allgemein für jede Periode des betrachteten Vorganges der Anfangs- und der Endpunkt des Vorganges oder verschiedener Teilvorgänge in Bezug auf deren zeitliches Auftreten wie auch daraus abgeleitet in Bezug auf deren elektrisches Potential einwandfrei fest. Es ist naheliegend, daß dieses Verfahren sich insbesondere zur Auswertung der ermittelten Daten in einer Datenverarbeitungsanlage oder einem Rechner eignet.

## Patentansprüche

1. Verfahren zur Bestimmung des Anfangs- und Endpunktes von sich wiederholenden Vorgängen, im Körper eines Lebewesens, die durch Meßwerte die mittels Elektroden abgegriffen werden in Funktion von Raum und Zeit dargestellt werden, dadurch gekennzeichnet, daß ein, einem so dargestellten Vorgang (8, 9, 10, QRS, T, P) zugeordnetes, Zeitintervall (25 + 26) in einen ersten Abschnitt (22, 25) und einen zweiten Abschnitt (23, 26) aufgeteilt wird, daß Abstände (27a, 27b) bestimmt werden, zwischen Punkten (11a, 12a, 11b, 12b), die Meßwerte in Abtastzeitpunkten des ersten Abschnittes (22, 25) im Raum darstellen und Punkten (14a bis 19a, 14b bis 19b), die Meßwerte in Abtastzeitpunkten des zweiten Abschnittes (23, 26) im Raum darstellen, daß der kleinste dieser Abstände (27a, 27b) ermittelt wird und daß der Abtastzeitpunkt im ersten Abschnitt (22, 25) und der Abtastzeitpunkt im zweiten Abschnitt (23, 26) derjenigen Punkte, die den kleinsten Abstand zueinander aufweisen, als Anfangs und als Endpunkt (20, 21) des Vorganges betrachtet werden.

2. Verfahren gemäß Anspruch 1, dadurch gekennzeichnet, daß das Zeitintervall (25 + 26) durch einen Referenzpunkt (24, 13a, 13b) in den ersten und den zweiten Abschnitt (22, 25 und 23) 26) getrennt wird.

3. Verfahren gemäß Anspruch 1, dadurch gekennzeichnet, daß der erste und der zweite Abschnitt (22, 25 und 23, 26) durch einen Referenzpunkt getrennt werden, in welchem der Vorgang in seiner Darstellung im Raum einen mittleren Steigungswert aufweist.

4. Verfahren gemäß Anspruch 3, dadurch gekennzeichnet, daß der Referenzpunkt in erster Näherung durch Vorgabe eines bestimmten Steigungswertes bestimmt wird, wobei der Referenzpunkt dann erreicht ist, wenn der Vorgang den vorgegeb nen Steigungswert erreicht und daß fuer weitere Perioden des Vorganges mittlere Steigungswerte ermittelt und als neue Vorgabe verwendet werden.

5. Verfahren gemäß Anspruch 1, dadurch gekennzeichnet, daß die Abstände in Projektionen des räumlich dargestellten Vorganges in mehrere Ebenen bestimmt werden und zu einem räumlichen Abstand umgerechnet werden.

## Claims

1. Method of determining the starting point and the end point of repetitive processes, in the body of a living being, represented by measured values, picked up by means of electrodes, as a function of space and time, comprising the steps of subdividing a predetermined period of time (25+26) which is associated with a represented process (8, 9, 10, QRS, T, P) into a first time interval (22, 25) and a second time interval (23, 26), determining the distances (27a, 27b) between points (11a, 12a, 11b, 12b) representing measured values in space picked up in sampling moments associated with said first time interval (22, 25) and points (14a to 19a, 14b to 19b) representing measured values in space picked up in sampling moments associated with said second time interval (23, 26), ascertaining the minimum value of said distances (27a, 27b) and defining a selected point associated with said first time interval (22, 25) and a selected point associated with said second time interval (23, 26), between which said minimum distance exists, as said starting point and said end point (20, 21) of the process.

2. Method according to claim 1, whereas said predetermined period of time (25+26) is subdivided into said first and said second time interval (22, 25 and 23, 26) by means of a reference point (24, 13a, 13b).

3. Method according to claim 1, whereas said first and said second time intervals (22, 25 and 23, 26) are separated by a reference point at which the spatial slope of the process has an average value.

4. Method according to claim 3, whereas said reference point is, in a first approximation, determined by the moment of time in which the spatial slope has reached a predetermined value, and whereas during other periods of the process, average values of the spatial slope are determined and used for determining new reference points.

5. Method according to claim 1, whereas said distances are determined in projections onto a plurality of planes of the spatial representation of the process and whereas a spatial distance is computed from said distances in said planes.

## Revendications

1. Procédé de détermination du point de départ et du point final de processus qui se répètent, qui ont lieu dans le corps d'un être vivant et qui sont représentés dans l'espace et le temps par des valeurs prises par des électrodes, caractérisé en ce qu'un intervalle de temps (25+26) associé à un processus (8, 9, 10, QRS, T, P) ainsi représenté est partagé en une première partie (22, 25) et une seconde partie (23, 26), que des distances (27a, 27b) sont établies entre des points (11a, 12a, 11b, 12b) qui constituent des valeurs mesurées dans l'espace en des temps d'échantillonnage appartenants à la première partie (22, 25) et des points (14a à 19a, 14b à 19b) qui constituent des valeurs mesurées dans l'espace en des temps d'échantillonnage appartenants à la seconde partie (23, 26), que la plus courte parmi ces distances (27a, 27b) est trouvée et en ce que le temps d'échantillonnage de la première partie (22, 25) et le temps d'échantillonnage de la seconde partie (23, 26) correspondant aux points qui ont la distance la plus courte sont considérés comme point de départ (20) et point final (21) du processus.

2. Procédé selon la revendication 1, caractérisé en ce que l'intervalle de temps (25+26) est partagé en deux parties (22, 25 et 23, 26) par le point de référence (24, 13a, 13b).

3. Procédé selon la revendication 1, caractérisé en ce que la première et la seconde partie (22, 25 et 23, 26) sont partagées par un point de référence où le processus présente une pente moyenne dans sa représentation dans l'espace.

4. Procédé selon la revendication 3, caractérisé en ce que le point de référence est obtenu en première approximation en fixant une valeur qui représente la pente, en considérant comme point de référence le temps correspondant au point dont la pente atteint en premier la valeur fixée et en prenant pour nouvelle approximation des valeurs moyennes de pentes établies dans d'autres périodes.

5. Procédé selon la revendication 1, caractérisé en ce que les distances sont établies dans des projections du processus représenté dans l'espace sur plusieurs plans et ensuite transformées en distances dans l'espace.

Fig. 2

Fig. 1